# EUROPÄISCHE PATENTSCHRIFT

(11) **EP 0 490 198 B1**
(45) Veröffentlichungstag und Bekanntmachung des Hinweises auf die Patenterteilung: **28.02.1996**
(21) Anmeldenummer: 91120613.4
(22) Anmeldetag: 29.11.1991
(51) Int. Cl.: C07C 69/734, C07C 67/327, C07C 59/66, C07C 51/377, C07C 67/46, A61K 7/40

(54) **Verfahren zur Herstellung von 3-Arylacrylsäuren und ihren Derivaten**
Process for the preparation of 3-aryle acrylic acids and their derivatives
Procédé de préparation d'acides 3-aryle acryliques et de leurs dérivés

(30) Priorität: 13.12.1990 DE 4039782
(43) Veröffentlichungstag der Anmeldung: 17.06.1992
(73) Patentinhaber: BASF Aktiengesellschaft, D-67063 Ludwigshafen (DE)
(72) Erfinder: Huellmann, Michael, Dr., W-6140 Bensheim (DE); Gnad, Josef, Dr., W-6700 Ludwigshafen (DE); Becker, Rainer, Dr., W-6702 Bad Duerkheim (DE)

(56) Entgegenhaltungen:
- DE-A- 3 437 634
- GB-A- 1 023 176
- US-A- 4 785 133
- US-A- 4 827 021

## Beschreibung

Die vorliegende Erfindung betrifft ein verbessertes Verfahren zur Herstellung von 3-Arylacrylsäuren und ihren Derivaten der allgemeinen Formel I 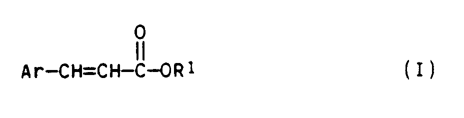
in der
- Ar: für einen Arylrest steht, welcher zusätzlich Substituenten tragen kann, die nicht mit Keten reagieren sowie unter den Bedingungen des erfindungsgemäßen Verfahrens säure- und basenstabil sind, und
- R¹: Wasserstoff, ein Alkalimetall, ein Erdalkalimetall, Ammonium oder eine C₁- bis C₂₀-Alkylgruppe bezeichnet.

Die bisherigen Herstellungsverfahren für die Verbindungen I basieren überwiegend auf basenkatalysierten Aldolreaktionen, und zwar beispielsweise ausgehend von aromatischen Aldehyden und Essigsäureestern. Nachteilig an dieser Methode ist die aufarbeitungsbedingt anfallende relativ hohe Salzmenge sowie die Gefahr der Bildung zahlreicher Nebenprodukte.

Eine andere bekannte Synthesemethode für die Verbindungen I basiert auf der Addition von Keten an aromatische Aldehyde. Beispielsweise arbeitet man hierbei gemäß der DE-A 34 37 634 (1) in Gegenwart katalytischer Mengen Eisen- und/oder Zinksalze von Mono- oder Dicarbonsäuren, wobei allerdings zur Bildung der freien Zimtsäuren ein aufwendiger Depolymerisationsschritt notwendig ist. Die Ausbeuten und die Reinheit der erhaltenen Produkte sind bei dem in (1) beschriebenen Verfahren jedoch noch verbesserungsbedürftig.

In der Literaturstelle Chem. Listy 47 (1953), 413-417, (2) wird die Herstellung von 3-Ethoxy-3-phenylpropionsäureethylester aus Benzaldehyddiethylacetal durch Umsetzung mit Keten in Gegenwart von 0,2 mol Bortrifluorid-Etherat bei 0°C beschrieben (siehe Tabelle 1). Die Ausbeute beträgt nur 32 %.

Die im erfindungsgemäßen Verfahren als Zwischenprodukte auftretenden 3-Arylpropionsäure-Derivate III sind bekannt, z.B. 3-Methoxy-3-phenylpropionsäuremethylester sowie sein p-Methyl-, p-Methoxy-, p-Chlor- und p-Fluorderivat aus der Literaturstelle J. Organomet. Chem. 234 (1982), 73-83, (3).

Aufgabe der vorliegenden Erfindung war es, ein im Hinblick auf Wirtschaftlichkeit, Ausbeute und Reinheit der Produkte verbessertes Herstellungsverfahren für die Verbindungen I bereitzustellen.

Demgemäß wurde ein verbessertes Verfahren zur Herstellung von 3-Arylacrylsäuren und ihren Derivaten der allgemeinen Formel I 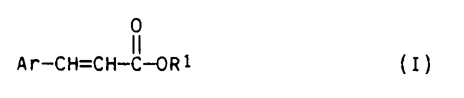
in der
- Ar: für einen Arylrest steht, welcher zusätzlich Substituenten tragen kann, die nicht mit Keten reagieren sowie unter den Bedingungen des erfindungsgemäßen Verfahrens säure- und basenstabil sind, und
- R¹: Wasserstoff, ein Alkalimetall, ein Erdalkalimetall, Ammonium oder eine C₁- bis C₂₀-Alkylgruppe bezeichnet,
gefunden, welches dadurch gekennzeichnet ist, daß man in einer ersten Reaktionsstufe ein Dialkylacetal eines aromatischen Aldehyds der allgemeinen Formel II 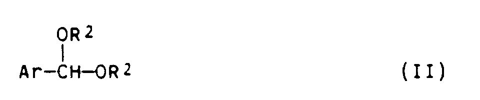
in der R² für C₁- bis C₄-Alkyl steht, mit Keten der Formel CH₂=C=O in Gegenwart katalytischer Mengen einer Protonen- oder einer Lewis-Säure zu einem 3-Arylpropionsäure-Derivat der allgemeinen Formel III 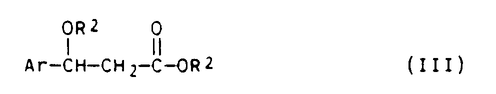
umsetzt und dieses Zwischenprodukt III in einer zweiten Reaktionsstufe in Gegenwart von Säure oder Base und zusätzlich für den Fall, daß R¹ eine C₁- bis C₂₀-Alkylgruppe bezeichnet, mit einem C₁- bis C₂₀-Alkanol zum Endprodukt I umsetzt.

In einer bevorzugten Ausführungsform steht Ar für einen Phenyl-, Biphenylyl- oder Naphthylrest, der durch ein bis drei C₁-C₄-Alkylgruppen, C₁-C₄-Alkoxygruppen, Hydroxylgruppen, Phenoxygruppen, Aminogruppen, welche durch C₁-C₄-Alkylgruppen mono- oder disubstituiert sein können, Halogenatome, Nitrogruppen oder eine Methylendioxygruppe substituiert sein kann, wobei die Substituenten gleich oder verschieden sein können.

Als Beispiele für Ar sind zu nennen:
Phenyl,
o-, m- oder p-Tolyl,
o-, m- oder p-Ethylphenyl,
o-, m- oder p-Propylphenyl,
m- oder p-Cumyl,
o-, m- oder p-Butylphenyl,
m- oder p-iso-Butylphenyl,
m- oder p-sec.-Butylphenyl,
m- oder p-tert.-Butylphenyl,
2,3-, 2,4-, 2,5-, 2,6-, 3,4- oder 3,5-Dimethylphenyl,
Mesityl,
o-, m- oder p-Methoxyphenyl,
o-, m- oder p-Ethoxyphenyl,
o-, m- oder p-Propoxyphenyl,
m- oder p-iso-Propoxyphenyl,
o-, m- oder p-Butoxyphenyl,
m- oder p-iso-Butoxyphenyl,
m- oder p-sec.-Butoxyphenyl,
m- oder p-tert.-Butoxyphenyl,
2,3-, 2,4-, 2,5-, 2,6-, 3,4- oder 3,5-Dimethoxyphenyl,
o-, m- oder p-Hydroxyphenyl,
2,3-, 2,4-, 2,5-, 2,6-, 3,4- oder 3,5-Dihydroxyphenyl,
3-Hydroxy-4-methoxyphenyl,
m- oder p-Phenoxyphenyl,
o-, m- oder p-Aminophenyl,
o-, m- oder p-(N-Methylamino)phenyl,
o-, m- oder p-(N,N-Dimethylamino)phenyl,
o-, m- oder p-Fluorphenyl,
o-, m- oder p-Chlorphenyl,
2,4-Dichlorphenyl,
o-, m- oder p-Bromphenyl,
o-, m- oder p-Nitrophenyl,
2,3- oder 3,4-Methylendioxyphenyl,
2-, 3- oder 4-Biphenylyl und
α- oder β-Naphthyl.

Besonders bevorzugt werden C₁-C₄-Alkoxyphenylreste, insbesondere, wenn der Alkoxyrest in p-Position am Phenylkern steht.

Bezeichnet der Rest R¹ Wasserstoff, ein Alkalimetall, vor allem Natrium oder Kalium, ein Erdalkalimetall, z.B. Calcium oder Magnesium, oder Ammonium, handelt es sich bei den Verbindungen I um die freien 3-Arylacrylsäuren bzw. deren Salze. Steht R¹ für eine C₁- bis C₂₀-Alkylgruppe, liegen 3-Arylacrylsäurealkylester vor. Bevorzugt werden hiervon Wasserstoff und insbesondere C₁- bis C₂₀-Alkylgruppen.

Beispiele für geradkettige oder verzweigte C₁- bis C₂₀-Alkylgruppen sind Methyl, Ethyl, n-Propyl, iso-Propyl, n-Butyl, tert.-Butyl, n-Pentyl, n-Hexyl, n-Heptyl, n-Octyl, 2-Ethylhexyl, n-Nonyl, iso-Nonyl, n-Decyl, n-Undecyl, n-Dodecyl, n-Tridecyl, iso-Tridecyl, n-Tetradecyl, n-Hexadecyl, n-Octadecyl und n-Eicosyl. Hiervon werden geradkettige oder verzweigte C₆- bis C₁₆-Alkylgruppen, insbesondere geradkettige oder verzweigte C₈- bis C₁₂-Alxylgruppen, bevorzugt. Von besonderem Interesse sind geradkettige oder verzweigte C₈-Alkylgruppen.

In der ersten Reaktionsstufe wird ein Dialkylacetal eines aromatischen Aldehyds II, bei dem als Acetalreste R² für C₁- bis C₄-Alkyl z.B. Methyl, Ethyl, n-Propyl, iso-Propyl, n-Butyl, 1,2-Ethylen oder 1,3-Propylen auftreten können, wobei Methyl und Ethyl bevorzugt werden, mit Keten üblicherweise bei einer Temperatur von -20 bis 80°C, insbesondere -10 bis 50°C, umgesetzt. Man setzt zweckmäßigerweise 0,8 bis 1,2 mol, vorzugsweise 0,9 bis 1,1 mol, insbesondere 0,94 bis 1,06 mol Keten pro Mol II ein.

Die Umsetzung wird vorzugsweise lösungsmittelfrei durchgeführt, kann aber auch in einem inerten protonenfreien organischen Lösungsmittel vorgenommen werden. Als derartige Lösungsmittel kommen Kohlenwasserstoffe, z.B. Pentan, Hexan, Heptan, Octan, Cyclohexan, Toluol, Xylol oder deren Gemische, oder Ether, z.B. Diethylether, Methyl-tert.-butylether oder Tetrahydrofuran, oder Mischungen hieraus in Betracht.

Die Umsetzung von II mit Keten wird in Gegenwart katalytischer Mengen einer Protonensäure, z.B. Schwefelsäure, Salzsäure, Essigsäure, Ameisensäure oder p-Toluolsulfonsäure, oder einer Lewis-Säure, z.B. eines Bortrihalogenid-Dialkyletherates, wie Bortrifluorid-Dimethyletherat oder Bortrifluorid-Diethyletherat, einer Titantetraalkoxyverbindung oder eines Zinkcarboxylates, durchgeführt. Unter katalytischen Mengen sind bei Protonensäuren üblicherweise Mengen von 0,001 bis 1 mol-% pro Mol II, bei Lewis-Säuren üblicherweise Mengen von 0,1 bis 10 mol-% pro Mol II zu verstehen.

Man arbeitet zweckmäßigerweise so, daß man eine Lösung der Protonensäure bzw. Lewis-Säure in der Ausgangsverbindung II, gegebenenfalls unter Zusatz von organischem Lösungsmittel, auf die Umsetzungstemperatur einstellt und das Keten, vorzugsweise drucklos, eingast. Das Keten reagiert in der Regel sofort ab. Da die Umsetzungen meist stark exotherm sind, empfiehlt es sich, die Reaktionswärme durch Kühlung abzuführen. Oft treten durch Spuren von Nebenprodukten intensive Färbungen der Reaktionsmischung auf, beispielsweise Violettfärbungen, was sich aber weiter nicht störend auswirkt.

Das Zwischenprodukt III läßt sich in der Regel leicht in reiner Form isolieren, beispielsweise durch Destillation im Vakuum. In einer bevorzugten Ausführungsform des erfindungsgemäßen Verfahrens wird jedoch auf die Isolierung des Zwischenproduktes III verzichtet und die weitere Umsetzung der zweiten Reaktionsstufe mit dem Rohprodukt der ersten Reaktionsstufe durchgeführt.

In der zweiten Reaktionsstufe wird das Zwischenprodukt III in Gegenwart von Säure, üblicherweise einer Protonensäure, z.B. Schwefelsäure Salzsäure, Essigsäure, Ameisensäure oder p-Toluolsulfonsäure, oder Base, z.B. Alkali- oder Erdalkalimetallhydroxid wie Natriumhydroxid, Kaliumhydroxid oder Calciumhydroxid oder Alkali- oder Erdalkalimetallalkoholat wie Natriummethylat, Natriumethylat oder Kalium-tert.-butylat, umgesetzt. Normalerweise reichen für derartige Hydrolyse- und Eliminierungsreaktionen übliche katalytische Mengen hiervon aus; will man aber beispielsweise das Alkali- oder Erdalkalimetallsalz einer 3-Arylacrylsäure I erhalten, sind stöchiometrische Mengen der entsprechenden Base notwendig.

Sollen 3-Arylacrylsäure-C₁- bis C₂₀-alkylester als 3-Arylacrylsäure-Derivate I hergestellt werden, muß zusätzlich ein C₁- bis C₂₀-Alkanol eingesetzt werden. Für die Auswahl der entsprechenden C₁- bis C₂₀-Alkylreste dieser Alkanole gilt das oben bei der Auswahl von R¹ Gesagte. Bei dieser Umsetzung handelt es sich um eine Umesterungsreaktion, d.h. einen Austausch der Alkylgruppe R² gegen eine Alkylgruppe R¹, bei gleichzeitiger Eliminierung eines zweiten Alkoholmoleküls R²-OH. Der C₁- bis C₂₀-Alkanol wird in äquimolarer Menge oder vorzugsweise im Überschuß, etwa 1,1 bis 10 mol pro Mol III, insbesondere 2 bis 10 mol pro Mol III, eingesetzt.

Bei der Durchführung der zweiten Reaktionsstufe arbeitet man in der Regel bei erhöhten Temperaturen, etwa bei 70 bis 250°C, insbesondere bei 100 bis 200°C. Bei 70 bis 100°C setzt die Eliminierung eines Äquivalentes R²-OH ein, bei 150 bis 250°C erfolgt die Hydrolyse- bzw. Umesterungsreaktion unter Abspaltung eines zweiten Äquivalentes R²-OH. Ein Überschuß an eingesetztem C₁- bis C₂₀-Alkanol wird üblicherweise abdestilliert. Das so erhaltene Rohprodukt wird zweckmäßigerweise nach den üblichen Methoden, beispielsweise durch Destillation im Vakuum, gereinigt.

Trägt die Gruppe Ar Substituenten, welche durch eine Säurebehandlung während des erfindungsgemäßen Verfahrens protoniert worden sind oder ein Addukt mit einer Lewis-Säure gebildet haben, oder Substituenten, welche durch eine Basenbehandlung während des erfindungsgemäßen Verfahrens anionisiert worden sind, können diese Substituenten leicht durch Behandeln mit einer entsprechenden Menge einer geeigneten Base oder Säure wieder in die ursprüngliche Form zurückgeführt werden.

Das erfindungsgemäße Verfahren eröffnet einen einfachen und wirtschaftlichen Weg zur Herstellung von 3-Arylacrylsäuren und ihren Derivaten I, zumal die Ausgangsverbindung II leicht durch elektrochemische Oxidation der Methylgruppe der entsprechenden Methylaromaten zugänglich ist. Die als Ausgangsverbindungen eingesetzten aromatischen Aldehyde des aus (1) bekannten Verfahrens können dagegen nur durch aufwendige Syntheseverfahren, beispielsweise durch die zudem toxikologisch bedenkliche Chromsäureoxidation von Methylaromaten, beispielsweise Toluol-Derivaten, erhalten werden.

Das erfindungsgemäße Verfahren liefert die Verbindungen I in guten Ausbeuten und frei von größeren Mengen an störenden Nebenprodukten, so daß sich anschließende Reinigungsoperationen unproblematisch gestalten. Insbesondere bei einer lösungsmittelfreien Fahrweise ergeben sich hervorragende Raum-Zeit-Ausbeuten.

Bei geschickter Wahl der Katalysatorsysteme in beiden Reaktionsstufen ist sogar die gleichzeitige Herstellung weiterer wirtschaftlich verwertbarer Produkte als Koppelprodukte mit den Verbindungen I möglich. Setzt man in der ersten Stufe beispielsweise ein Bortrifluorid-Dialkyletherat als Katalysator und in der zweiten Stufe ein Natriumalkoholat als Katalysator ein, kann man zusätzlich Natriumfluorid und einen Borsäuretrialkylester aus dem Verfahren gewinnen.

### Beispiel

### Herstellung von p-Methoxyzimtsäure-2-ethylhexylester

In eine Mischung aus 364 g (2,0 mol) p-Methoxybenzaldehyd-dimethylacetal und 10 ml (0,11 mol) Bortrifluorid-Dimethyletherat wurden bei ca. 15°C innerhalb von 3 Stunden 89 g (2,12 mol) Keten eingegast, wobei die freiwerdende Reaktionswärme durch Kühlung mit Eiswasser abgeführt wurde. Bei der Ketenzugabe färbte sich die Reaktionslösung tief violett. Nach beendeter Keteneinleitung erhielt man 458 g einer violetten Flüssigkeit, die nach gaschromatographischer Analyse 76,1 Gew.-% an 3-Methoxy-3-(p-methoxyphenyl)-propionsäuremethylester enthielt.

Diese Flüssigkeit wurde mit 40 g einer 30 gew.-%igen methanolischen Natriummethylat-Lösung versetzt, wobei sich ein pH-Wert von ca. 10 einstellte und die violette in eine gelbbraune Farbe umschlug. Nach Zugabe von 780 g (6,0 mol) 2-Ethylhexanol wurde die Mischung langsam auf 150 bis 180°C erhitzt, wobei innerhalb von ca. 1 Stunde 151 g einer hauptsächlich aus Methanol bestehenden Flüssigkeit abdestillierten. Überschüssiges 2-Ethylhexanol wurde abdestilliert und der erhaltene Rückstand im Ölpumpenvakuum bei ca. 1 mbar destillativ gereinigt. Man erhielt 436 g der Titelverbindung mit einem Siedepunkt von 165°C bei 0,3 mbar, entsprechend einer Ausbeute von 75 %, bezogen auf eingesetztes p-Methoxybenzaldehyddimethylacetal.

## Patentansprüche

1. Verfahren zur Herstellung von 3-Arylacrylsäuren und ihren Derivaten der allgemeinen Formel I 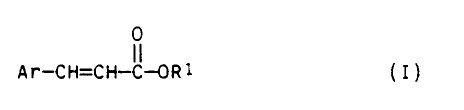 in der
Ar für einen Arylrest steht, welcher zusätzlich Substituenten tragen kann, die nicht mit Keten reagieren sowie unter den Bedingungen der unten beschriebenen Umsetzung säure- und basenstabil sind, und
R¹ Wasserstoff, ein Alkalimetall, ein Erdalkalimetall, Ammonium oder eine C₁- bis C₂₀-Alkylgruppe bezeichnet,
dadurch gekennzeichnet, daß man in einer ersten Reaktionsstufe ein Dialkylacetal eines aromatischen Aldehyds der allgemeinen Formel II 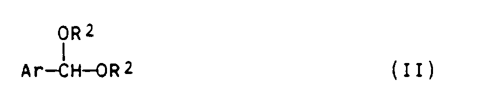 in der R² für C₁- bis C₄-Alkyl steht, mit Keten der Formel CH₂=C=O in Gegenwart katalytischer Mengen einer Protonen- oder einer Lewis-Säure zu einem 3-Arylpropionsäure-Derivat der allgemeinen Formel III 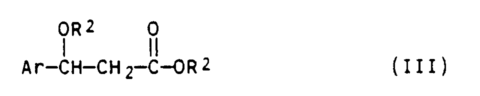 umsetzt und dieses Zwischenprodukt III in einer zweiten Reaktionsstufe in Gegenwart von Säure oder Base und zusätzlich für den Fall, daß R² eine C₁- bis C₂₀-Alkylgruppe bezeichnet, mit einem C₁- bis C₂₀-Alkanol zum Endprodukt I umsetzt.

2. Verfahren nach Anspruch 1, dadurch gekennzeichnet, daß man es auf die Herstellung von 3-Arylacrylsäuren und ihren Derivaten I anwendet, bei denen
Ar für einen Phenyl-, Biphenylyl- oder Naphthylrest steht, der durch ein bis drei C₁-C₄-Alkylgruppen, C₁-C₄-Alkoxygruppen, Hydroxylgruppen, Phenoxygruppen, Aminogruppen, welche durch C₁-C₄-Alkylgruppen mono- oder disubstituiert sein können, Halogenatome, Nitrogruppen oder eine Methylendioxygruppe substituiert sein kann, wobei die Substituenten gleich oder verschieden sein können.

3. Verfahren nach Anspruch 1 oder 2, dadurch gekennzeichnet, daß man es ohne Isolierung des Zwischenproduktes III durchführt.

## Claims

1. A process for preparing a 3-arylacrylic acid or its derivative of the formula I 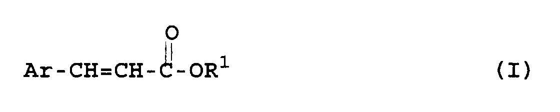 where
Ar is aryl which can additionally have substituents which do not react with ketene and are stable under the conditions of the reaction described below, and
R¹ is hydrogen, an alkali metal, an alkaline earth metal, ammonium or C₁-C₂₀-alkyl,
which comprises a first stage in which a dialkyl acetal of an aromatic aldehyde of the formula II 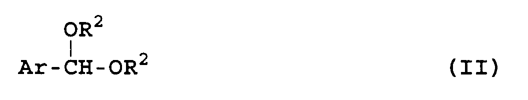 where R² is C₁-C₄-alkyl, is reacted with ketene of the formula CH₂=C=O in the presence of catalytic amounts of a protic or Lewis acid to give a 3-arylpropionic acid derivative of the formula III 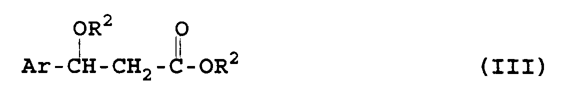 and a second stage in which this intermediate III is reacted in the presence of acid or base and, in the case where R² is C₁-C₂₀-alkyl, additionally with a C₁-C₂₀-alkanol to give the final product I.

2. A process as claimed in claim 1, which is used to prepare a 3-arylacrylic acid or its derivative I, where
Ar is phenyl, biphenylyl or naphthyl, each of which can be substituted by one to three C₁-C₄-alkyl groups, C₁-C₄-alkoxy groups, hydroxyl groups, phenoxy groups, amino groups which can be mono- or disubstituted by C₁-C₄-alkyl, or halogen atoms, nitro groups or a methylenedioxy group, it being possible for the substituents to be identical or different.

3. A process as claimed in claim 1 or 2, which is carried out without isolation of the intermediate III.

## Revendications

1. Procédé de préparation d'acides 3-arylacryliques et leurs dérivés de formule générale I 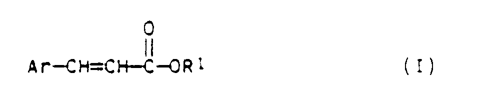 dans laquelle
Ar est mis pour un reste aryle qui peut porter additionnellement des substituants qui ne réagissent pas avec une cétine, et également sont stables aux acides et aux bases dans les conditions de la réaction décrite ci-dessous
R¹ signifie hydrogène, un métal alcalin, un métal alcalino-tèrreux, ammonium ou un groupe alkyle en C1 à C20
caractérisé par le fait que, dans une première étape de réaction, on fait réagir un dialkylacétal d'un aldéhyde aromatique de formule II 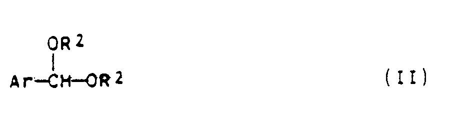 dans laquelle R² est mis pour alkyle en C1 à C4 avec une cétine de formule CH₂ = C = O en présence de quantités catalytiques d'un acide à protons ou un acide de Lewis pour aboutir à un dérivé d'acide 3-arylpropionique de formule générale III 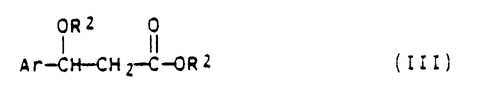 et dans une deuxième étape de réaction on fait réagir ce produit intermédiaire III en présence d'acide ou de base et éventuellement, dans le cas où R² représente un groupe alkyle en C1 à C20, avec un alcanol en C1 à C20 pour aboutir au produit final I.

2. Procédé selon la revendication 1, caractérisé par le fait qu'il est utilisé à la préparation d'acides 3-arylacryliques et leurs dérivés, dans lesquels
Ar est mis pour un reste phényle, biphénylyle ou naphtyle, qui peut être substitué par un à trois groupes alkyle en C1-C4, groupes alcoxy en C1-C4, groupes hydroxyle, groupes phénoxy, groupes amino, qui peuvent être mono- ou disubstitués par des groupes alkyle en C1-C4, des atomes halogène, des groupes nitro ou un groupe méthylènedioxy, les substituants pouvant être identiques ou différents.

3. Procédé selon la revendication 1 ou 2, caractérisé par le fait qu'on l'effectue sans isolement du produit intermédiaire III.
